(19) Europäisches Patentamt

European Patent Office

Office européen des brevets

(11) **EP 0 599 019 B1**

(12) **EUROPÄISCHE PATENTSCHRIFT**

(45) Veröffentlichungstag und Bekanntmachung des Hinweises auf die Patenterteilung:
**07.03.2001   Patentblatt 2001/10**

(51) Int Cl.[7]: **C07D 211/46**, B01J 23/86

(21) Anmeldenummer: **93115531.1**

(22) Anmeldetag: **25.09.1993**

(54) **Verfahren zur Herstellung von 4-Hydroxy-2.2.6.6-tetramethylpiperidin**

A process for the preparation of 4-hydroxy-2,2,6,6-tetramethylpiperidine

Un procédé de préparation de la 4-hydroxy-2,2,6,6-tétraméthylpipéridine

(84) Benannte Vertragsstaaten:
**BE CH DE ES FR GB IT LI NL**

(30) Priorität: **21.11.1992   DE 4239247**

(43) Veröffentlichungstag der Anmeldung:
**01.06.1994   Patentblatt 1994/22**

(73) Patentinhaber: **Degussa-Hüls Aktiengesellschaft
60287 Frankfurt am Main (DE)**

(72) Erfinder:
• **Thelen, Gerhard, Dr.
D-48301 Nottuln (DE)**
• **Mock, Guthard
D-45768 Marl (DE)**
• **Hallmann, Otto
D-45770 Marl (DE)**

(56) Entgegenhaltungen:
**EP-A- 0 290 387          EP-A- 0 405 958
US-A- 4 208 539**

**Beschreibung**

Die vorliegende Erfindung beschreibt ein Verfahren zur Hydrierung von 4-Oxo-2.2.6.6-tetra-methylpiperidin (Triacetonamin, TAA) zu 4-Hydroxy-2.2.6.6-tetramethylpiperidin (TAA-ol). Die verwendeten Katalysatoren können als Festbett angeordnet in kontinuierlichen Verfahren und als Pulver in Batchverfahren diskontinuierlich eingesetzt werden.

Aus der Literatur sind zahlreiche Katalysatoren für die Herstellung von 4-Hydroxy-2.2.6.6-tetramethylpiperidin bekannt. So ist z.B. in CS-PS 235 486, CS-PS 235 487 und CS-PS 236 437 der Einsatz von Nickelkatalysatoren beschrieben. Das TAA kommt jeweils als alkoholische Lösung, z. B. in Isopropanol und Isobutanol, zum Einsatz. In weiteren Patentschriften sind andere Katalysator-Lösungsmittel-Kombinationen erwähnt. Ciba Geigy schlägt in der DE-A 26 56 765 den Einsatz von Ni- und Edelmetallkatalysatoren in Lösungsmitteln wie Toluol, Xylol und Ether, in der DE-A 26 56 764 Ni und Ru als Katalysator und Wasser als Lösungsmittel, in der DE-A 26 56 763 Ni und Edelmetalle (Ru, Rh, Os, Ir, Pt) als Katalysatormetalle und tri-n-Alkylphosphate als Lösungsmittel, in der EP-A 225 850 und der EP-A 375 612 Ru als Katalysator und Wasser oder Alkohole als Lösungsmittel vor. Schließlich beansprucht die EP-A 290 387 ein Hydrierverfahren in Gegenwart eines Metallkatalysators aus der Gruppe der Ni-, Co-, Ru-, Rh-, Os- und Ir-Katalysatoren in Abwesenheit eines Lösungsmittels. Bevorzugt werden Ni- und Ru-Katalysatoren.

Vom ökologischen und ökonomischen Standpunkt aus ist es erforderlich, daß die Hydrierung von TAA zum TAA-ol bei vollständigem Umsatz und sehr hoher Selektivität verläuft. Eingesetzte Lösungsmittel können, da nur ein sehr geringer Anteil Nebenprodukte entsteht, vollständig recycliert werden. Die Verluste an TAA-ol bleiben gering.

Es wurde nun überraschenderweise gefunden, daß die Hydrierung von TAA zu TAA-ol in Gegenwart von Kupferkatalysatoren mit sehr hoher Selektivität verläuft, so daß die bekannten Nachteile, wie z.B. die aufwendige Abtrennung von Nebenprodukten, die Abwasserbelastung und der Produkt- und Lösungsmittelverlust, verhindert werden können. Dieser Vorteil tritt besonders dann in Erscheinung, wenn die Hydrierung in Abwesenheit eines Lösungsmittels durchgeführt wird.

Gegenstand der Erfindung ist daher ein Verfahren zur Herstellung von 4-Hydroxy-2.2.6.6-tetramethylpiperidin durch heterogenkatalytische Hydrierung von 4-Oxo-2.2.6.6-tetramethylpiperidin in Schmelze oder Lösung bei Temperaturen von 60 °C bis 200 °C und Wasserstoffdrücken von 10 bis 300 bar, welches dadurch gekennzeichnet ist, daß der Katalysator

20 bis 68 Gew.-% Kupfer,
0 bis 40 Gew.-% Chrom,
0 bis 60 Gew.-% Zink,
0 bis 5 Gew.-% Mangan,
0 bis 12 Gew.-% Barium,
0 bis 15 Gew.-% Silicium,
0 bis 10 Gew.-% Nickel

- jeweils bezogen auf die oxidische Katalysatormasse - enthält und mit Wasserstoff oder einem Wasserstoff enthaltenden Gasgemisch aktiviert worden ist.

Die Kupferkatalysatoren sind durch Zusatz weiterer Metalle aktiviert. Bevorzugt werden Cr, Mo, W, Mn, Re, Fe, Co, Ni, Zn, Ba und Si. Erfindungsgemäß werden die Kupferkatalysatoren in den üblichen Formen eingesetzt, d.h. die katalytisch aktiven Metalle kommen als sogenannte Vollkontakte in Granulat-, Extrudat- oder Tablettenform oder auf Träger aufgebracht als Trägerkatalysatoren zum Einsatz. Als Träger kommen handelsübliche unlösliche Stoffe, wie z. B. Aktivkohle, Kieselgur, Aluminiumoxid usw. in Frage. Für die kontinuierliche Hydrierung ist die bevorzugte Form Tabletten mit den Abmessungen 3 mm x 3 mm bis zu Größen von 9 mm x 9 mm, insbesondere 3 mm x 3 mm bis 6 mm x 6 mm sowie Extrudate mit einem Durchmesser von 1 mm bis 5 mm. Die bevorzugte Form für die batchweise Hydrierung ist der Einsatz der Kupfer-Katalysatoren als Pulver.

Die Kupferkatalysatoren enthalten 20 % bis 68 % Kupfer. Als Promotor wird Chrom in einem Bereich von 0 % bis 40 %, Mangan in einem Bereich von 0 % bis 5 %, Barium in einem Bereich von 0 % bis 12 %, Silicium in einem Bereich von 0 % bis 15 %, Nickel in einem Bereich von 0 % bis 10 % und Zink in einem Bereich von 0 % bis 60 % - jeweils bezogen auf die oxidische Katalysatormasse - zugesetzt. In einer bevorzugten Ausführungsform setzt sich der Katalysator aus 26 % bis 41 % Kupfer, 24 % bis 38 % Chrom, 0 % bis 4 % Mangan und 0 % bis 12 % Barium - bezogen auf die oxidische Katalysatormasse - zusammen. Nach der BET-Methode beträgt die spezifische Oberfläche 10 bis 60 $m^2$/g. Das Porenvolumen liegt zwischen 0,1 bis 0,5 $cm^3$/g.

In einer weiteren bevorzugten Ausführungsform setzt sich der Kupferkatalysator aus 20 % bis 65 % Kupfer und 20 % bis 60 % Zink - jeweils bezogen auf die oxidische Katalysatormasse - zusammen. Vorzugsweise enthält der Katalysator 22 bis 28 % Kupfer und 49 bis 55 % Zink. Die spezifische Oberfläche (BET) beträgt 30 bis 70 $m^2$/g, das Porenvolumen 0,2 bis 0,5 $cm^3$/g.

Die Kupferkatalysatoren in Form von Trägerkatalysatoren enthalten neben 30 % bis 80 % Träger, 10 % bis 40 % Kupfer, 0 % bis 15 % Chrom, 0 % bis 5 % Barium und 0 % bis 20 % Eisen. Die nach der BET-Methode bestimmte spezifische Oberfläche beträgt 10 bis 250 $m^2$/g. Das Porenvolumen liegt zwischen 0,1 bis 0,9 $cm^3$/g. Als bevorzugtes Trägermaterial wird Kieselgel und Aluminiumoxid eingesetzt.

Die Hydrierung wird zweckmäßig bei Temperaturen von 60 ° C bis 200 ° C, vorzugsweise von 80 °C bis 180 °C, durchgeführt. Bei diskontinuierlicher Hydrierung startet man die Reaktion vorzugsweise bei niedriger Temperatur, insbesondere von 60 bis 80 °C. Während des Reaktionsfortganges steigert man die Temperatur auf 120 bis 150 °C, insbesondere 130 bis 150 °C, und hält sie konstant.

Die kontinuierliche Hydrierung wird bei konstanter Temperatur im bevorzugten Bereich von 120 bis 180 °C, insbesondere 140 bis 170 °C, durchgeführt. In diesem Falle setzt man die Kupferkatalysatoren als Festbett ein, es entfällt die Filtration des Katalysators. Durch die hohe Selektivität, die durch die Kupferkatalysatoren erreicht wird, ist das TAA-ol sehr rein. Die Raum-Zeit-Ausbeute ist bei Verwendung der Kupferkatalysatoren gegenüber den bisher bekannten Katalysatoren und Verfahrensbeschreibungen wesentlich erhöht.

Die Umsetzung von TAA erfolgt in Gegenwart der Kupferkatalysatoren bei Wasserstoffdrücken von 10 bis 300 bar. Bei diskontinuierlicher Hydrierung wird Wasserstoff im Druckbereich von 10 bis 100 bar, bevorzugt bei 20 bis 95 bar, eingesetzt. Die kontinuierliche Herstellung von TAA-ol wird in Druckbereichen von 10 bis 300 bar durchgeführt. Die Anlagen im Hochdruckbereich arbeiten üblicherweise bei 150 bis 300 bar. Gleichfalls ist die Umsetzung von TAA zu TAA-ol ebenfalls bei 10 bis 100 bar, bevorzugt bei 20 bis 95 bar, mit den genannten Vorteilen durchführbar.

Die Hydrierung von TAA zu TAA-ol erfolgt unter den genannten Bedingungen in Gegenwart eines Alkohols als Lösungsmittel, wie z. B. Isobutanol, n-Butanol, 2-Ethylhexanol. Vorteilhaft wird die Umsetzung von TAA in Abwesenheit eines Lösungsmittels durchgeführt.

Die Hydrierzeit schwankt, sie ist abhängig vom Katalysator, von der Katalysatorkonzentration, vom Wasserstoffdruck und von der Reaktionstemperatur. Sie kann von 1 Minute bis 2 Stunden betragen. Bei Verwendung der Katalysatoren als Festbett sind Volumengeschwindigkeiten LHSV (liquid hourly space velocity) von 0,5 bis 3 $h^{-1}$, insbesondere 0,5 bis 1,5 $h^{-1}$, zu erwarten, wobei LHSV als

$$LHSV = \frac{\text{Eduktmenge in 1/h}}{\text{Katalysatormenge in 1}}$$

definiert ist.

Im diskontinuierlichen Verfahren setzt man den Katalysator in einer Menge von 0,1 bis 3 %, vorzugsweise 0,1 bis 2 %, insbesondere von 0,2 bis 1,5 %, bezogen auf das eingesetzte TAA, ein.

4-Hydroxy-2.2.6.6-tetramethylpiperidin ist ein bedeutendes Zwischenprodukt zur Herstellung von Stabilisatoren aus der Klasse der sterisch gehinderten Amine.

Die nachfolgenden Beispiele sollen die Erfindung erläutern.

### **Katalyatorbeispiele**

#### Beispiel 1

Der Katalysatortyp I enthielt 36 Gew.-% Kupfer, 32 Gew.-% Chrom, 2,5 Gew.-% Barium und 2,5 Gew.-% Mangan. Die Oberfläche betrug ca. 30 $cm^2$/g, eingesetzt wurden Tabletten mit den Maßen 3 x 3 mm.

#### Beispiel 2

Im Katalysatortyp II waren 27 Gew.-% Kupfer, 25 Gew.-% Chrom und 7 Gew.-% Barium enthalten. Der Katalysator in Tablettenform von 1/8" x 1/8" besaß eine Oberfläche von ca. 30 $m^2$/g.

#### Beispiel 3

Beispielhaft für einen CuZn-Typ (Katalysatortyp III) wurde eine oxidische Masse, bestehend aus 26 Gew.-% Kupfer und 53 Gew.-% Zink in Form von 3 x 3 mm Tabletten getestet. Der Katalysator besaß eine Oberfläche von 45 $m^2$/g.

#### Beispiel 4

Der Trägerkatalysator Typ IV enthielt neben 57 Gew.-% Kieselgel als Trägermaterial 23,0 Gew.-% Kupfer und 4,2 Gew.-% Eisen. Die oxidische Masse lag als Extrudat mit 1,2 mm Durchmesser und einer BET-Oberfläche von 210 $m^2$/g vor.

Beispiel 5

Ein Katalysatortyp V der chemischen Zusammensetzung 62 Gew.-% $SiO_2$, 21,3 Gew.-% Kupfer, 4,5 Gew.-% Barium und 2,0 Gew.-% Chrom besaß als Extrudat mit 4 mm Durchmesser eine BET-Oberfläche von 130 $m^2$/g

Beispiel 6

Der Typ VI bestand aus 40,8 Gew.-% Kupfer und 31,6 Gew.-% Chrom, jeweils bezogen auf das oxidische Gemisch. Die Oberfläche des Pulverkatalysators betrug 42 $m^2$/g.

Beispiel 7

Der Pulverkatalysator (Typ VII) setzte sich aus 36,5 Gew.-% Kupfer und 32 Gew.-% Chrom sowie 0,5 Gew.-% Barium und 4 Gew.-% Mangan zusammen. Die Oberfläche betrug 30 $m^2$/g.

**Beispiele**

Kontinuierliche Hydrierung

Eine Testanlage für Hochdruck-Hydrierungen bestand aus einem $H_2$-Druck-Ventil, einer Vorlage für TAA, einer Förderpumpe für TAA, einem 400 ml großen Reaktor und einem Trennbehälter zur Isolierung des Produktes. Der Reaktor enthielt jeweils 400 ml des Kupferkatalysators. Die gesamte Apparatur stand unter einem $H_2$-Druck von p bar, der Reaktor wurde bei einer Temperatur T betrieben. Das erhaltene Produkt wurde gaschromatographisch analysiert.
In der nachfolgenden Tabelle sind Ergebnisse mit verschiedenen Katalysatoren aufgelistet.

| Beispiel | Katalysatortyp | Reaktionsdruck p (bar) | Reaktionstemperatur T (°C) | LHSV ($h^{-1}$) | TAA Umsatz (mol-%) | Selektivität TAA-ol (mol-%) |
|---|---|---|---|---|---|---|
| 1 | I | 300 | 150 | 1 | > 99,9 | > 99 |
| 2 | | 300 | 170 | 1 | > 99,9 | > 99 |
| 3 | | 150 | 170 | 0,8 | > 99,9 | > 99 |
| 4 | II | 300 | 170 | 1 | > 99,9 | > 99 |
| 5 | III | 300 | 170 | 1 | > 99,9 | > 99 |
| 6 | | 150 | 170 | 1 | > 99,9 | > 99 |
| 7 | IV | 300 | 170 | 0,5 | > 99 | > 99 |
| 8 | V | 300 | 160 | 0,8 | > 99 | > 99 |

Diskontinuierliche Hydrierung

Für die diskontinuierliche batchweise Hydrierung stand ein 1 lgroßer Autoklav zur Verfügung. Zur Durchführung des Versuches wurden 400 ml TAA vorgelegt, eine vorgegebene Menge des Pulverkatalysators zugesetzt, auf Reaktionstemperatur $T_1$ aufgeheizt, ein Reaktionsdruck p eingestellt und abschließend bei einer Temperatur von $T_2$ gehalten. Das Produkt wurde nach dem Entspannen über eine Fritte vom Katalysator getrennt und gaschromatographisch analysiert.

| Beispiel | Katalysatortyp | Reaktionsdruck p (bar) | Reaktionstemperatur | | Konzentr. Kat. (g/l) | TAA Umsatz (mol-%) | Selektiv. TAA-ol (mol-%) |
|---|---|---|---|---|---|---|---|
| | | | $T_1$ (°C) | $T_2$ (°C) | | | |
| 9 | VI | 90 | 70 | 150 | 10 | > 99 | > 99 |
| 10 | VII | 90 | 80 | 150 | 12 | > 99 | > 99 |

**Beispiel 11**

Eine Lösung, bestehend aus 50 Teilen TAA und 50 Teilen Isobutanol, wurde kontinuierlich an einem Festbettkatalysator vom Typ I bei einer Temperatur von 145 °C, einem Reaktionsdruck von 300 bar und bei einer LHSV = 0,8 h$^{-1}$ hydriert. Die gaschromatographische Analyse des Produktes ergab einen TAA-Umsatz > 99,9 mol-% und eine Selektivität zu TAA-ol von > 99 mol.-%.

**Patentansprüche**

1. Verfahren zur Herstellung von 4-Hydroxy-2.2.6.6-tetramethylpiperidin durch heterogenkatalytische Hydrierung von 4-Oxo-2.2.6.6-tetramethyl-piperidin in Schmelze oder Lösung bei Temperaturen von 60 °C bis 200 °C und Wasserstoffdrücken von 10 bis 300 bar,
   dadurch gekennzeichnet,
   daß der Katalysator

   20 bis 68 Gew.-% Kupfer,
   0 bis 40 Gew.-% Chrom,
   0 bis 60 Gew.-% Zink,
   0 bis 5 Gew.-% Mangan,
   0 bis 12 Gew.-% Barium,
   0 bis 15 Gew.-% Silicium,
   0 bis 10 Gew.-% Nickel

   - jeweils bezogen auf die oxidische Katalysatormasse - enthält und mit Wasserstoff oder einem Wasserstoff enthaltenden Gasgemisch aktiviert worden ist.

2. Verfahren nach Anspruch 1,
   dadurch gekennzeichnet,
   daß der Katalysator - bezogen auf die oxidische Masse -

   26 bis 41 Gew.-% Kupfer
   24 bis 38 Gew.-% Chrom
   0 bis 4 Gew.-% Mangan
   0 bis 12 Gew.-% Barium

   enthält.

3. Verfahren nach Anspruch 1,
   dadurch gekennzeichnet,
   daß der Katalysator - bezogen auf die oxidische Masse

   20 bis 65 Gew.-% Kupfer
   20 bis 60 Gew.-% Zink

   enthält.

4. Verfahren nach Anspruch 1,
   dadurch gekennzeichnet,
   daß der Katalysator - bezogen auf die oxidische Masse -

   22 bis 28 Gew.-% Kupfer
   49 bis 55 Gew.-% Zink

   enthält.

5. Verfahren nach Anspruch 1,
   dadurch gekennzeichnet,
   daß die Kupferkatalysatoren auf Trägern aufgebracht sind und

   30 bis 80 Gew.-% Trägermaterial

10 bis 40 Gew.-% Kupfer
0 bis 15 Gew.-% Chrom
0 bis 5 Gew.-% Barium
0 bis 20 Gew.-% Eisen

enthalten.

6. Verfahren nach Anspruch 5,
dadurch gekennzeichnet,
daß als Trägermaterial Aktivkohle, Kieselgel oder Aluminiumoxid eingesetzt wird.

**Claims**

1. A process for the preparation of 4-hydroxy-2,2,6,6-tetramethylpiperidine by heterogeneous catalytic hydrogenation of 4-oxo-2,2,6,6-tetramethyl-piperidine in a melt or solution at temperatures of 60 °C to 200 °C and hydrogen pressures of 10 to 300 bar, characterised in that the catalyst contains

20 to 68 % by weight of copper,
0 to 40 % by weight of chromium,
0 to 60 % by weight of zinc,
0 to 5 % by weight of manganese,
0 to 12 % by weight of barium,
0 to 15 % by weight of silicon,
0 to 10 % by weight of nickel

- in each case based on the oxidic catalyst mass - and has been activated by hydrogen or a hydrogen-containing gas mixture.

2. A process according to claim 1, characterised in that the catalyst - based on the oxidic mass - contains

26 to 41 % by weight of copper
24 to 38 % by weight of chromium
0 to 4 % by weight of manganese
0 to 12 % by weight of barium.

3. A process according to claim 1, characterised in that the catalyst - based on the oxidic mass - contains

20 to 65 % by weight of copper
20 to 60 % by weight of zinc.

4. A process according to claim 1, characterised in that the catalyst - based on the oxidic mass - contains

22 to 28 % by weight of copper
49 to 55 % by weight of zinc.

5. A process according to claim 1, characterised in that the copper catalysts are applied to supports and contain

30 to 80 % by weight of support material
10 to 40 % by weight of copper
0 to 15 % by weight of chromium
0 to 5 % by weight of barium
0 to 20 % by weight of iron.

6. A process according to claim 5, characterised in that activated charcoal, silica gel or aluminium oxide is used as the support material.

**Revendications**

1. Procédé de production de 4-hydroxy 2,2,6,6 tétraméthyl-pipéridine par hydrogénation catalytique en phase hétérogène de 4-oxo 2,2,6,6-tétraméthylpiperidine à l'état fondu ou en solution à des températures de 60° C à 200°C et à des pressions d'hydrogène de 10 à 300 bars,
caractérisé en ce que
le catalyseur renferme

   de 20 à 68 % en poids de cuivre
   de 0 à 40 % en poids de chrome
   de 0 à 60 % en poids de zinc
   de 0 à 5 % en poids de manganèse
   de 0 à 12 % en poids de baryum
   de 0 à 15 % en poids de silicium
   de 0 à 10 % en poids de nickel

à chaque fois rapporté à la masse de catalyseur oxydique, et a été activé avec l'hydrogène ou un mélange gazeux contenant de l'hydrogène.

2. Procédé selon la revendication 1,
caractérisé en ce que
le catalyseur renferme - rapporté à la masse oxydique -

   de 26 à 41 % en poids de cuivre
   de 24 à 38 % en poids de chrome
   de 0 à 4 % en poids de manganèse
   de 0 à 12 % en poids de baryum.

3. Procédé selon la revendication 1,
caractérisé en ce que
le catalyseur renferme, rapporté à la masse oxydique,

   de 20 à 65 % en poids de cuivre
   de 20 à 60 % en poids de zinc.

4. Procédé selon la revendication 1,
caractérisé en ce que
le catalyseur renferme, rapporté à la masse oxydique,

   de 22 à 28 % en poids de cuivre
   de 49 à 55 % en poids de zinc.

5. Procédé selon la revendication 1,
caractérisé en ce que
les catalyseurs en cuivre sont appliqués sur des supports et renferment

   de 30 à 80 % en poids de matériau de support
   de 10 à 40 % en poids de cuivre
   de 0 à 15 % en poids de chrome
   de 0 à 5 % en poids de baryum
   de 0 à 20 % en poids de fer.

6. Procédé selon la revendication 5,
caractérisé en ce que
comme matériau du support on utilise du charbon activé, du gel de silice ou de l'oxyde d'aluminium.